# EUROPEAN PATENT APPLICATION

(11) **EP 2 647 381 A1**
(43) Date of publication of application: **09.10.2013**
(21) Application number: 11844056.9
(22) Date of filing: 02.12.2011
(51) Int. Cl.: A61K 31/616, A61K 9/24, A61K 9/26, A61K 31/4439, A61K 47/04, A61K 47/32, A61K 47/36, A61K 47/44, A61P 1/04, A61P 9/10, A61P 29/00, A61P 43/00

(54) **ORALLY DISINTEGRATING TABLET**

(30) Priority: 03.12.2010 JP 2010270276
(71) Applicant: Takeda Pharmaceutical Company Limited, Osaka-shi, Osaka 541-0045 (JP)
(72) Inventor: MIMA, Yasushi, Osaka-shi Osaka 532-0024 (JP); KAWANO, Tetsuya, Osaka-shi Osaka 532-0024 (JP); MIZUNO, Yumiko, Osaka-shi Osaka 532-0024 (JP)
(74) Representative: Kingsbury, Oliver William
(86) International application number: PCT/JP2011/077978
(87) International publication number: WO 2012/074110

(57) **Abstract**

The present invention relates to a multi-layer orally disintegrating tablet having (1) an enteric fine granule-containing layer containing a proton pump inhibitor and (2) an acetylsalicylic acid-containing layer, which shows high stability of the active ingredients (proton pump inhibitor, aspirin) and expresses the pharmacological effects of the active ingredients stably and rapidly after administration.

## Description

### Technical Field

The present invention relates to an orally disintegrating tablet comprising a proton pump inhibitor (hereinafter sometimes referred to as PPI) and acetylsalicylic acid (hereinafter sometimes referred to as aspirin). More particularly, the present invention relates to an orally disintegrating tablet superior in the stability of the active ingredient, which expresses a pharmacological effect stably and rapidly after the administration.

### (Background of the Invention)

Low-dose aspirin administered for the purpose of suppressing formation of thrombus and embolus (antiplatelet therapy) in the diseases of the brain blood vessel and circulatory organ region sometimes causes gastric ulcer or duodenal ulcer. Since discontinuation of aspirin administration may cause formation of thrombus or embolus, it is considered important to continue administration of low-dose aspirin while suppressing the onset of ulcer. Aspirin is also known as a non-steroidal anti-inflammatory drug (NSAIDs), and mainly used for the treatment of pain, fever and inflammation. However, NSAIDs may cause gastric ulcer or duodenal ulcer. Particularly, in the treatment of rheumatoid arthritis, osteoarthritis and the like, since QOL decreases markedly, discontinuation of NSAIDs administration is sometimes difficult. Therefore, it is considered important to continue NSAIDs administration while suppressing the onset of ulcer.
On the other hand, PPIs of benzimidazole compounds such as lansoprazole, omeprazole and the like are widely used as therapeutic agents for peptic ulcer and the like, since they have a strong gastric acid secretion-inhibitory action, a gastric mucosa protecting action and the like. Particularly, a lansoprazole preparation has been approved to show the efficacy of "suppression of the onset of gastric ulcer or duodenal ulcer in administration of low-dose aspirin" and "suppression of the onset of gastric ulcer or duodenal ulcer in administration of non-steroidal anti-inflammatory drug" also in Japan in recent years, and the clinical effect of the suppression of the onset of gastric ulcer or duodenal ulcer caused by aspirin medication has been verified.
Patent document 1 discloses a pharmaceutical composition containing (a) a therapeutically effective amount of at least one proton pump inhibitor unstable to acid; (b) a sufficient amount of at least one buffering agent to increase pH of the gastric fluid to a pH that prevents acid decomposition of at least some proton pump inhibitors in the gastric fluid; and (c) a therapeutically effective amount of at least one non-steroidal anti-inflammatory drug.
Patent document 2 discloses an oral dosage form containing an acid sensitive proton pump inhibitor together with at least one kind of a non-steroidal anti-inflammatory drug (NSAID) and, when desired, a pharmaceutically acceptable excipient.
Patent document 3 discloses a drug composition in a unit dosage form suitable for oral administration to patients, (a) an acid suppressant present in an amount effective for increasing the gastric pH of the aforementioned patients to at least 3.5 after administration of one kind or a plurality of the aforementioned unit dosage form, and (b) a non-steroidal anti-inflammatory drug in an amount effective for decreasing or removing a pain or inflammation in the aforementioned patients after administration of one kind or a plurality of the aforementioned unit dosage form, wherein the aforementioned unit dosage form cooperatively releases the aforementioned NSAID after the aforementioned acid suppressant.
Furthermore, while PPI such as lansoprazole and the like or aspirin is already commercially available each other as single agent, an orally disintegrating tablet containing both PPI and acetylsalicylic acid (combination drug of layered tablet) is not known.

### [Document List]

### [patent documents]

patent document 1: WO2005/076987
patent document 2: WO97/25064
patent document 3: WO2002/098352

### SUMMARY OF THE INVENTION

### Problems to be Solved by the Invention

Provision of a preparation containing both PPI and acetylsalicylic acid as active ingredients (combination drug) has extremely high clinical usefulness. However, practicalization of a preparation containing plural active ingredients is not easy compared to that of a preparation containing a single active ingredient. For example, since the dissolution rate of the active ingredient from a preparation can affect the time-course drug efficacy profile after administration, it is necessary in the practicalization of a preparation to control the composition of the preparation so that the dissolution rate of the active ingredient can be optimized. In the case of a combination drug, however, the dissolution rate of each active ingredient needs to be optimized, which is highly difficult from the aspect of pharmaceutical technology. It is also necessary to suppress the adverse influence (degradation of the preservation or chemical stability such as time-course decomposition of the active ingredients, decreased activity of the active ingredients and the like, degradation of the dissolution stability such as time-course changes in the active ingredient dissolution pattern and the like, and the like) caused by an interaction of plural active ingredients contained in the combination drug.
Moreover, with the aging population and changes in life environment, it is desired to develop an orally disintegrating tablet capable of being administered easily, maintaining the convenience for handling which is a characteristic of a tablet, and being administered on demand easily, anytime and anywhere, without water.
The present inventors have conducted intensive studies and found that a multi-layer orally disintegrating tablet having (1) an enteric fine granule-containing layer comprising a proton pump inhibitor and (2) an acetylsalicylic acid-containing layer shows high stability of the active ingredients and permits stable and rapid expression of a pharmacological effect of the active ingredient after administration, which resulted in the completion of the present invention. Means of Solving the Problems

Accordingly, the present invention provides
[1] a multi-layer orally disintegrating tablet comprising (1) an enteric fine granule-containing layer comprising a proton pump inhibitor and (2) an acetylsalicylic acid-containing layer,
[2] the orally disintegrating tablet of the aforementioned [1], wherein the enteric fine granule-containing layer comprises an antacid in a part other than the enteric fine granules,
[3] the orally disintegrating tablet of the aforementioned [2], wherein the antacid is at least one kind of component selected from the group consisting of metal oxide, metal hydroxide, alkaline earth metal carbonate and aluminum glycinate,
[4] the orally disintegrating tablet of the aforementioned [3], wherein the metal oxide is at least one kind selected from the group consisting of magnesium oxide, magnesium silicate, dried aluminum hydroxide gel and magnesium aluminometasilicate,
[5] the orally disintegrating tablet of the aforementioned [3], wherein the metal hydroxide is at least one kind selected from the group consisting of magnesium hydroxide, aluminum hydroxide, synthetic hydrotalcite, coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate,
[6] the orally disintegrating tablet of the aforementioned [3], wherein the alkaline earth metal carbonate is calcium carbonate or magnesium carbonate,
[7] the orally disintegrating tablet of the aforementioned [2], wherein the antacid is a mixture of magnesium carbonate and aluminum glycinate,
[8] the orally disintegrating tablet of the aforementioned [2], wherein the content of the antacid is about 10 mg - about 100 mg,
[9] the orally disintegrating tablet of the aforementioned [1], wherein the proton pump inhibitor is lansoprazole, omeprazole, rabeprazole, pantoprazole or an optically active form thereof or a salt thereof,
[10] the orally disintegrating tablet of the aforementioned [1], wherein the content of the acetylsalicylic acid is about 70 mg - about 120 mg per tablet,
[11] the orally disintegrating tablet of the aforementioned [1], wherein the acetylsalicylic acid-containing layer comprises carboxymethylcellulose,
[12] the orally disintegrating tablet of the aforementioned [1], wherein the enteric fine granule-containing layer comprising a proton pump inhibitor comprises at least one kind of a disintegrant selected from crospovidone and magnesium aluminometasilicate in a part other than the enteric fine granules,
[13] the orally disintegrating tablet of the aforementioned [1], wherein the acetylsalicylic acid-containing layer comprises a lubricant,
[14] the orally disintegrating tablet of the aforementioned [13] wherein the lubricant is hydrogenated oil,
[15] the orally disintegrating tablet of the aforementioned [1], which is a bi-layer tablet,
[16] the orally disintegrating tablet of the aforementioned [1], comprising an intermediate layer between (1) the enteric fine granule-containing layer comprising a proton pump inhibitor and (2) the acetylsalicylic acid-containing layer,
[17] the orally disintegrating tablet of the aforementioned [1], wherein the acetylsalicylic acid is not enteric-coated,
[18] the orally disintegrating tablet of the aforementioned [1], wherein the tablet weight is about 300 mg - about 800 mg, and the weight ratio of (1) the enteric fine granule-containing layer comprising a proton pump inhibitor and (2) the acetylsalicylic acid-containing layer is about 10:1 - about 1:10,
[19] the orally disintegrating tablet of the aforementioned [1], having a curved surface on a tablet surface,
[20] the orally disintegrating tablet of the aforementioned [1], having an oral disintegration time of within about 60 seconds, and
[21] the orally disintegrating tablet of the aforementioned [1], having a tablet hardness of about 20 - about 100 N.

### Effect of the Invention

Since the orally disintegrating tablet of the present invention contains PPI having a strong acid secretion-inhibitory action and acetylsalicylic acid useful as an agent for the prophylaxis or treatment of diseases in the brain blood vessel or circulatory organ region, for example, angina pectoris (chronic stable angina pectoris, unstable angina pectoris), an inhibitor of thrombus or embolus formation in myocardial infarction; an agent for the prophylaxis or treatment of ischemic cerebrovascular disorders (transient cerebral ischemic attack (TIA), cerebral infarction); an inhibitor of thrombus or embolus formation after operation of coronary artery bypass graft (CABG) or percutaneous transluminal coronary angioplasty (PTCA); or an agent for the prophylaxis or treatment of Kawasaki disease (including cardiovascular sequelae due to Kawasaki disease), the orally disintegrating tablet of the present invention can be administered for the purpose of treating or suppressing the onset of gastric ulcer or duodenal ulcer while continuing the administration of aspirin.
In addition, since acetylsalicylic acid can be used mainly for the treatment of pain, fever and inflammation as one kind of a non-steroidal anti-inflammatory drug (NSAIDs), the orally disintegrating tablet of the present invention can also be administered for the purpose of treating or suppressing the onset of gastric ulcer or duodenal ulcer while continuing the administration of NSAIDs.
The orally disintegrating tablet of the present invention shows high stability of the active ingredient, and expresses the pharmacological effect of the active ingredient stably and rapidly after administration.
The orally disintegrating tablet of the present invention can be administered easily while maintaining the convenience for handling, and can be administered on demand easily, anytime and anywhere, without water.

### (Detailed Description of the Invention)

The present invention is explained in detail in the following.
The solid preparation of the present invention comprises (1) an enteric fine granule-containing layer comprising a proton pump inhibitor and (2) an acetylsalicylic acid-containing layer in combination, and is particularly a multi-layer orally disintegrating tablet.

### (1) "enteric fine granule-containing layer comprising PPI"

### (1)-1: PPI

Examples of the PPI is preferably a compound represented by the following formula (I) [hereinafter sometimes simply referred to as compound (I)].

As compound (I), a compound represented by the formula (I) :

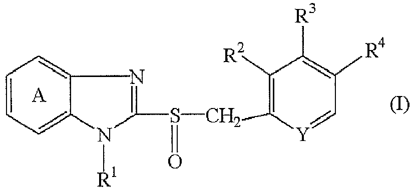

wherein
ring A is a benzene ring optionally having substituent(s),
R¹ is a hydrogen atom, an aralkyl group optionally having substituent(s), an acyl group or an acyloxy group,
R², R³ and R⁴ are the same or different and each is a hydrogen atom, an alkyl group optionally having substituent(s), an alkoxy group optionally having substituent(s) or an amino group optionally having substituent(s), and
Y is a nitrogen atom or CH,
or an optically active form thereof or a salt thereof can be mentioned.

In the above-mentioned compound (I), examples of the "substituent" of the "benzene ring optionally having substituent(s)" for ring A include a halogen atom, a cyano group, a nitro group, an alkyl group optionally having substituent(s), a hydroxy group, an alkoxy group optionally having substituent(s), an aryl group, an aryloxy group, a carboxy group, an acyl group, an acyloxy group, a 5- to 10-membered heterocyclic group and the like. The benzene ring optionally has 1 to 3 of these substituents. When the number of the substituents is not less than 2, respective substituents may be the same or different. Of these substituents, a halogen atom, an alkyl group optionally having substituent(s), an alkoxy group optionally having substituent(s) and the like are preferable.
Examples of the halogen atom include fluorine, chlorine, bromine atom and the like. Of these, fluorine atom is preferable.
Examples of the "alkyl group" of the "alkyl group optionally having substituent(s)" include a C₁₋₇ alkyl group (e.g., a methyl, ethyl, propyl, isopropyl, butyl, isobutyl, sec-butyl, tert-butyl, pentyl, hexyl, heptyl group etc.). Examples of the "substituent" of the "alkyl group optionally having substituent(s)" include a halogen atom, a hydroxy group, a C₁₋₆ alkoxy group (e.g., methoxy, ethoxy, propoxy, butoxy etc.), a C₁₋₆ alkoxy-carbonyl group (e.g., methoxycarbonyl, ethoxycarbonyl, propoxycarbonyl etc.), a carbamoyl group and the like. The number of the substituents may be 1 to 3. When the number of the substituents is not less than 2, respective substituents may be the same or different.
Examples of the "alkoxy group" of the "alkoxy group optionally having substituent(s)" include a C₁₋₆ alkoxy group (e.g., methoxy, ethoxy, propoxy, isopropoxy, butoxy, isobutoxy, pentoxy etc.) and the like. Examples of the "substituent" of the "alkoxy group optionally having substituent(s)" include those similar to the "substituent" of the above-mentioned "alkyl group optionally having substituent(s)". The number of the substituents is the same as in the above-mentioned "alkyl group optionally having substituent(s)".
Examples of the "aryl group" include a C₆₋₁₄ aryl group (e.g., phenyl, 1-naphthyl, 2-naphthyl, biphenyl, 2-anthryl etc.) and the like.
Examples of the "aryloxy group" include a C₆₋₁₄ aryloxy group (e.g., phenyloxy, 1-naphthyloxy, 2-naphthyloxy etc.) and the like.
Examples of the "acyl group" include formyl, alkylcarbonyl, alkoxycarbonyl, carbamoyl, alkylcarbamoyl, alkylsulfinyl, alkylsulfonyl and the like.
Examples of the "alkylcarbonyl group" include a C₁₋₆ alkyl-carbonyl group (e.g., an acetyl, propionyl etc.) and the like.
Examples of the "alkoxycarbonyl group" include a C₁₋₆ alkoxy-carbonyl group (e.g., methoxycarbonyl, ethoxycarbonyl, propoxycarbonyl, butoxycarbonyl etc.) and the like.
Examples of the "alkylcarbamoyl group" include an N-C₁₋₆ alkyl-carbamoyl group (e.g., methylcarbamoyl, ethylcarbamoyl group etc.), an N,N-di-C₁₋₆ alkyl-carbamoyl group (e.g., N,N-dimethylcarbamoyl, N,N-diethylcarbamoyl etc.) and the like.
Examples of the "alkylsulfinyl group" include a C₁₋₇ alkylsulfinyl group (e.g., methylsulfinyl, ethylsulfinyl, propylsulfinyl, isopropylsulfinyl etc.) and the like.
Examples of the "alkylsulfonyl group" include a C₁₋₇ alkylsulfonyl group (e.g., methylsulfonyl, ethylsulfonyl, propylsulfonyl, isopropylsulfonyl etc.) and the like.
Examples of the "acyloxy group" include alkylcarbonyloxy, alkoxycarbonyloxy, carbamoyloxy, alkylcarbamoyloxy, alkylsulfinyloxy, alkylsulfonyloxy and the like.
Examples of the "alkylcarbonyloxy group" include a C₁₋₆ alkyl-carbonyloxy group (e.g., acetyloxy, propionyloxy etc.) and the like.
Examples of the "alkoxycarbonyloxy group" include a C₁₋₆ alkoxy-carbonyloxy group (e.g., methoxycarbonyloxy, ethoxycarbonyloxy, propoxycarbonyloxy, butoxycarbonyloxy etc.) and the like.
Examples of the "alkylcarbamoyloxy group" include a C₁₋₆ alkyl-carbamoyloxy group (e.g., methylcarbamoyloxy, ethylcarbamoyloxy etc.) and the like.
Examples of the "alkylsulfinyloxy group" include a C₁₋₇ alkylsulfinyloxy group (e.g., methylsulfinyloxy, ethylsulfinyloxy, propylsulfinyloxy, isopropylsulfinyloxy etc.) and the like.
Examples of the "alkylsulfonyloxy group" include a C₁₋₇ alkylsulfonyloxy group (e.g., methylsulfonyloxy, ethylsulfonyloxy, propylsulfonyloxy, isopropylsulfonyloxy etc.) and the like.
Examples of the "5- to 10-membered heterocyclic group" include a 5- to 10-membered (preferably 5- or 6-membered) heterocyclic group containing, besides carbon atoms, one or more (e.g., 1 to 3) heteroatoms selected from a nitrogen atom, a sulfur atom and an oxygen atom. Specific examples include a 2- or 3-thienyl group, a 2-, 3- or 4-pyridyl group, a 2- or 3-furyl group, a 1-, 2- or 3-pyrrolyl group, a 2-, 3-, 4-, 5- or 8-quinolyl group, a 1-, 3-, 4- or 5-isoquinolyl group, a 1-, 2-or 3-indolyl group and the like. Of these, a 5- or 6-membered heterocyclic group such as a 1-, 2- or 3-pyrrolyl group is preferable.
Ring A is preferably a benzene ring optionally having 1 or 2 substituents selected from a halogen atom, an optionally halogenated C₁₋₄ alkyl group, an optionally halogenated C₁₋₄ alkoxy group and 5- or 6-membered heterocyclic group.

Examples of the "aralkyl group" of the "aralkyl group optionally having substituent(s)" for R¹ include a C₇₋₁₆ aralkyl group (e.g., a C₆₋₁₀ aryl-C₁₋₆ alkyl group such as benzyl, phenethyl etc.) and the like. Examples of the "substituent" of the "aralkyl group optionally having substituent(s)" include those similar to the "substituent" of the above-mentioned "alkyl group optionally having substituent(s)". The number of the substituents is 1 to 4. When the number of the substituents is not less than 2, respective substituents may be the same or different.
Examples of the "acyl group" for R¹ include those similar to the "acyl group" exemplified as the substituent of the above-mentioned ring A.
Examples of the "acyloxy group" for R¹ include those similar to the "acyloxy group" exemplified as the substituent of the above-mentioned ring A.
R¹ is preferably a hydrogen atom.

Examples of the "alkyl group optionally having substituent(s)" for R², R³ or R⁴ include those similar to the "alkyl group optionally having substituent(s)" exemplified as the substituent of the above-mentioned ring A.
Examples of the "alkoxy group optionally having substituent(s)" for R², R³ or R⁴ include those similar to the "alkoxy group optionally having substituent(s)" exemplified as the substituent of the above-mentioned ring A.
Examples of the "amino group optionally having substituent(s)" for R², R³ or R⁴ include an amino group, a mono-C₁₋₆ alkylamino group (e.g., methylamino, ethylamino etc.), a mono-C₆₋₁₄ arylamino group (e.g., phenylamino, 1-naphthylamino, 2-naphthylamino etc.), a di-C₁₋₆ alkylamino group (e.g., dimethylamino, diethylamino etc.), a di-C₆₋₁₄ arylamino group (e.g., diphenylamino etc.) and the like.
R² is preferably a C₁₋₆ alkyl group, a C₁₋₆ alkoxy group, a C₁₋₆ alkoxy-C₁₋₆ alkoxy group or a di-C₁₋₆ alkylamino group. R² is more preferably a C₁₋₃ alkyl group or a C₁₋₃ alkoxy group.
R³ is preferably a hydrogen atom, a C₁₋₆ alkoxy-C₁₋₆ alkoxy group or an optionally halogenated C₁₋₆ alkoxy group. R³ is more preferably a C₁₋₃ alkoxy group which is halogenated or optionally substituted by a C₁₋₃ alkoxy group.
R⁴ is preferably a hydrogen atom or a C₁₋₆ alkyl group. R⁴ is more preferably a hydrogen atom or a C₁₋₃ alkyl group (it is particularly preferably a hydrogen atom).
Y is preferably a nitrogen atom.

A preferable compound of the formula (I) is a compound wherein ring A is a benzene ring optionally having substituent(s) selected from a halogen atom, an optionally halogenated C₁₋₄ alkyl group, an optionally halogenated C₁₋₄ alkoxy group and a 5- or 6-membered heterocyclic group, R¹ is a hydrogen atom, R² is a C₁₋₆ alkyl group, a C₁₋₆ alkoxy group, a C₁₋₆ alkoxy-C₁₋₆ alkoxy group or a di-C₁₋₆ alkylamino group, R³ is a hydrogen atom, a C₁₋₆ alkoxy-C₁₋₆ alkoxy group or an optionally halogenated C₁₋₆ alkoxy group, R⁴ is a hydrogen atom or a C₁₋₆ alkyl group, and Y is a nitrogen atom.

Of compounds (I), a compound represented by the formula (Ia):

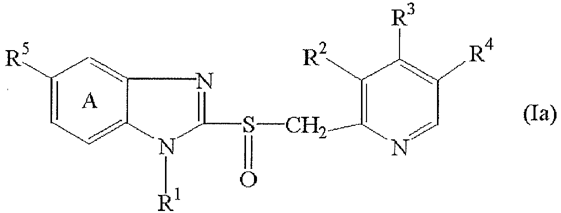

wherein R¹ is a hydrogen atom, R² is a C₁₋₃ alkyl group or a C₁₋₃ alkoxy group, R³ is a halogenated C₁₋₃ alkoxy group or a C₁₋₃ alkoxy group optionally substituted by a C₁₋₃ alkoxy group, R⁴ is a hydrogen atom or a C₁₋₃ alkyl group, R⁵ is a hydrogen atom, an optionally halogenated C₁₋₃ alkoxy group or a pyrrolyl group (e.g., 1-, 2- or 3-pyrrolyl group).
In the formula (Ia), a compound wherein R¹ is a hydrogen atom, R² is a C₁₋₃ alkyl group, R³ is an optionally halogenated C₁₋₃ alkoxy group, R⁴ is a hydrogen atom, R⁵ is a hydrogen atom or an optionally halogenated C₁₋₃ alkoxy group is particularly preferable.

Specific examples of compound (I) include the following compounds.
2-[[[3-Methyl-4-(2,2,2-trifluoroethoxy)-2-pyridinyl]methyl]sulfinyl]-1H-benzimidazole, 2-[[(3,5-dimethyl-4-methoxy-2-pyridinyl)methyl]sulfinyl]-5-methoxy-1H-benzimidazole(omeprazole), 2-[[[4-(3-methoxypropoxy)-3-methyl-2-pyridinyl]methyl]sulfinyl]-1H-benzimidazole(rabeprazole)· sodium salt, 5-difluoromethoxy-2-[[(3,4-dimethoxy-2-pyridinyl)methyl]sulfinyl]-1H-benzimidazole(pantoprazole) and the like.
Of these compounds, 2-[[[3-methyl-4-(2,2,2-trifluoroethoxy)-2-pyridinyl]methyl]sulfinyl]-1H-benzimidazole (lansoprazole) is preferable.

The compound (I) may be a racemate or an optically active form such as R-form and S-form. For example, compound (I) may be an optically active form such as (R)-2-[[[3-methyl-4-(2,2,2-trifluoroethoxy)-2-pyridinyl]methyl]sulfinyl]-1H-benzimidazole, and the optically active form is preferable.

The salt of compound (I) or an optically active form thereof is preferably a pharmaceutically acceptable salt, for example, a salt of compound (I) or an optically active form thereof with an inorganic base, a salt with an organic base, a salt with a basic amino acid and the like.
Preferable examples of the salt with inorganic base include alkali metal salts such as sodium salt, potassium salt and the like; alkaline earth metal salts such as calcium salt, magnesium salt and the like; ammonium salt and the like.
Preferable examples of the salt with organic base include salts with alkylamines (trimethylamine, triethylamine etc.), heterocyclic amines (pyridine, picoline etc.), alkanolamines (ethanolamine, diethanolamine, triethanolamine etc.), dicyclohexylamine, N,N'-dibenzylethylenediamine and the like.
Preferable examples of the salt with basic amino acid include salts with arginine, lysine, ornithine and the like.
Of these salts, alkali metal salts or alkaline earth metal salts are preferable. Sodium salt is particularly preferable.
The compound (I) can be produced according to a method known per se, for example, the method described in JP-A-S61-50978, US-B-4,628,098, JP-A-H10-195068, WO98/21201 or the like, or a method analogous thereto.
An optically active form of compound (I) can be obtained according to a method such as optical resolution (fractional recrystallization, chiral column method, diastereomer method, method using microorganism or enzyme, and the like), asymmetric oxidation and the like. For example, R form of lansoprazole can also be produced by the method described in WO00/78745, WO01/83473, WO01/87874 and WO02/44167.

PPI used in the present invention is preferably selected from benzimidazole compounds having an antiulcer activity such as lansoprazole, omeprazole, rabeprazole and pantoprazole, optically active forms thereof and pharmaceutically acceptable salts thereof.

### (1)-2: "enteric fine granules containing PPI"

The "enteric fine granules containing PPI" means fine granules having an average particle size of not more than about 400 µm, wherein a composition containing PPI is coated with an enteric coating layer.
The "coating" is used to mean not only coating the entire surface of the subject (e.g., core) to be coated, but also partially coating, being adsorbed or being absorbed. Unless otherwise specified, the "average particle size" means a volume median diameter (median diameter: a particle diameter corresponding to 50% of cumulative distribution). Examples of the measurement method thereof include a laser diffraction particle size distribution measuring method, specifically, a method using a laser diffraction particle size distribution analyzer HEROS RODOS (manufactured by Sympatec, Germany).

The "fine granules having an average particle size of not more than about 400 µm, wherein a composition containing PPI is coated with an enteric coating layer" have an average particle size of not more than about 400 µm to avoid rough texture and discomfort in the mouth. A preferable average particle size is 300 - 400 µm.
When the size of the maximum particles is defined rather than the average particle size of the "fine granules", the particle size is substantially not more than 425 µm, preferably substantially not more than 400 µm. The preferable range is a particle size of substantially 300 - 425 µm, and more preferably, substantially 300 - 400 µm.
The "substantially" in "particle size is substantially not more than 425 µm" and "particle size is substantially not more than 400 µm" means that a small amount (not more than about 5 wt%) of particles having a particle size outside each of the aforementioned ranges may be contained as long as they are unavoidably mixed.
In the present invention, the content of PPI in the "composition containing PPI" (composition before coating with an enteric coating layer) is, for example, not less than about 5 wt%, preferably about 10 - 50 wt%, more preferably about 15-50 wt%, particularly preferably about 20 - about 50 wt%.
The content of PPI in the whole preparation is, for example, not less than about 1 wt%, preferably not less than about 1.5 wt%, not more than 10.0 wt%, more preferably not less than about 2.0 wt%, not more than 8.0 wt%.

In the above-mentioned "composition", a basic inorganic salt is preferably blended to stabilize the physiologically active substance (PPI) in the preparation.
Examples of the "basic inorganic salt" include basic inorganic salts of sodium, potassium, magnesium and/or calcium. Preferred is a basic inorganic salt of magnesium and/or calcium. More preferred is a basic inorganic salt of magnesium.
Examples of the basic inorganic salt of sodium include sodium carbonate, sodium hydrogen carbonate and the like.
Examples of the basic inorganic salt of potassium include potassium carbonate, potassium hydrogen carbonate and the like.
Examples of the basic inorganic salt of magnesium include heavy magnesium carbonate, magnesium carbonate, magnesium oxide, magnesium hydroxide, magnesium aluminometasilicate, magnesium silicate, magnesium aluminate, synthetic hydrotalcite [Mg₆Al₂(OH)₁₆·CO₃·4H₂O] and magnesia alumina hydrate [2.5MgO· Al₂O₃·xH₂O], with preference given to heavy magnesium carbonate, magnesium carbonate, magnesium oxide, magnesium hydroxide and the like.
Examples of the basic inorganic salt of calcium include precipitated calcium carbonate, calcium hydroxide and the like.
As the "basic inorganic salt", more preferred are heavy magnesium carbonate, magnesium carbonate, magnesium oxide, magnesium hydroxide and the like.
These basic inorganic salts such as magnesium, calcium and the like may be any as long as a 1% aqueous solution or suspension thereof has a basic pH (not less than pH 7).
One or more kinds of the basic inorganic salt (preferably basic inorganic salts of magnesium, calcium) may be blended in combination, and the amount thereof can be appropriately selected according to the kind of the basic inorganic salt. The amount is, for example, about 0.3 - 200 wt%, preferably about 1 - 100 wt%, more preferably about 10 - 50 wt%, most preferably about 20 - 40 wt%, relative to PPI.

The "composition" may contain a water-soluble polymer, and the below-mentioned binder, lubricant, excipient and the like used for the production of general preparations. The amount to be added is an amount used for the production of general preparations.
Examples of the "water-soluble polymer" include ethanol-soluble water-soluble polymers [for example, cellulose derivatives such as hydroxypropylcellulose (hereinafter sometimes described as HPC) and the like, polyvinylpyrrolidone and the like], ethanol-insoluble water-soluble polymers [for example, cellulose derivatives such as hydroxypropylmethylcellulose (hereinafter sometimes described as HPMC), methylcellulose, sodium carboxymethylcellulose and the like, sodium polyacrylate, polyvinyl alcohol, sodium alginate, guar gum and the like] and the like.
When a water-soluble polymer is used, the dissolution property of a drug (PPI) can be controlled by concurrently using an ethanol-soluble water-soluble polymer and an ethanol-insoluble water-soluble polymer or using water-soluble polymers having different viscosity in combination.

In the present invention, preferable water-soluble polymer includes cellulose derivatives such as HPC, HPMC, methylcellulose and the like, polyvinyl alcohol, more preferably, cellulose derivatives such as HPC, HPMC and the like.
HPC contains a hydroxypropoxyl group at, for example, about 53.4 - 77.5 wt%, preferably about 60 - 70 wt%. A 2 wt% aqueous solution of HPC has viscosity at 20°C of generally about 1 - 150000 cps (centipoise). As such HPC, the Japanese Pharmacopoeia hydroxypropylcellulose and the like are used (hereinafter the viscosity of HPC is always that of 2 wt% aqueous solution at 20°C).
HPMC is mixed ether wherein methoxy group and hydroxypropoxy group are bonded. HPMC has a methoxy group content of, for example, about 19 - 30 wt%, and a hydroxypropoxy group content of, for example, about 4 - 12 wt%. A 2 wt% aqueous solution of HPMC has viscosity at 20°C of generally about 1 - 40000 centistokes. As such HPMC, hydroxypropylmethylcellulose 2208 defined by the Japanese Pharmacopoeia, hydroxypropylmethylcellulose 2906 defined by the Japanese Pharmacopoeia, hydroxypropylmethylcellulose 2910 defined by the Japanese Pharmacopoeia and the like are used. One kind of or a mixture of two or more kinds of hydroxypropylmethylcellulose can be used.
The content of the water-soluble polymer such as HPC and/or HPMC and the like is generally about 0.1 - 50 wt%, preferably about 1 - 30 wt%, since it can control the dissolution property of PPI in a composition containing the PPI, and maintains a high content of a physiologically active substance.

Examples of the "enteric coating layer" that covers the above-mentioned "composition containing PPI" include aqueous enteric polymer bases such as cellulose acetate phthalate (CAP), hydroxypropylmethylcellulose phthalate (hereinafter to be described as HP-55), hydroxymethylcellulose acetate succinate, methacrylic acid copolymer [for example, Eudragit L30D-55 (trade name; manufactured by Evonik), KollICoat MAE30DP (trade name; manufactured by BASF), Polyquid PA30 (trade name; manufactured by SanyoKasei) and the like], carboxymethylethylcellulose, shellac and the like; sustained-release bases such as methacrylate copolymer [for example, Eudragit NE30D (trade name), Eudragit RL30D (trade name), Eudragit RS30D (trade name) and the like] and the like; water-soluble polymer; plasticizers such as triethyl citrate, polyethylene glycol, acetylated monoglyceride, triacetine, castor oil and the like, and the like. One kind of or a mixture of two or more kinds of these may be used.
A preferable aqueous enteric polymer base is a methacrylic acid copolymer.
A preferable sustained-release base is a methacrylate copolymer, particularly a copolymer of ethylacrylate and methylmethacrylate.
The amount of the sustained-release base to be used is about 5 - 30 wt%, preferably about 5 - 15 wt%, per 100 wt% of the aqueous enteric polymer base. A preferable amount of the plasticizer to be used is 5 - 30 wt% per 100 wt% of the aqueous enteric polymer base.

The aforementioned "composition containing PPI" can be produced by a known granulation method.
The "granulation method" includes, for example, rotary granulation method (e.g., centrifugal rotary granulation), fluidized bed granulation method (e.g., rotary fluidized bed granulation, fluidized granulation, etc.), stirring granulation method and the like. Among others, preferred is fluidized bed granulation method, more preferred is rotary fluidized bed granulation method.
Concrete example of the rotary granulation method includes a method using "CF apparatus" manufactured by Freund Corporation and the like. Concrete examples of the rotary fluidized bed granulation method include methods using "SPIR-A-FLOW", "multi plex" manufactured by Powrex Corporation, "New-Marumerizer" manufactured by Fuji Paudal Co., Ltd., and the like. The method for spraying the mixture can be suitably selected in accordance with the kind of granulator, and may be, for example, any one of a top spray method, a bottom spray method, a tangential spray method, and the like. Among others, a tangential spray method is preferred.

The "composition containing PPI" is produced by, for example, coating a core containing crystalline cellulose and lactose with PPI.
For example, a method including coating a core containing crystalline cellulose and lactose with a physiologically active substance unstable to acid and, where necessary, a basic inorganic salt, a binder, a lubricant, an excipient, a water-soluble polymer and the like (hereinafter sometimes to be abbreviated as a coating layer), which is described in the production method (coating method) described in JP-A-H5-092918 and the like, can be mentioned. For example, a method including coating a core with a physiologically active substance unstable to acid and a basic inorganic salt, and then with a binder, a lubricant, an excipient, a water-soluble polymer and the like can be mentioned.

The average particle size of the "core" only needs to be not more than about 250 µm, and is about 50 - 250 µm, preferably about 100 - 250 µm, more preferably about 100 - 200 µm. The cores having the above-described average particle size include particles all of which pass through a No. 50 (300 µm) sieve, about 5 w/w% or less of the entirety of which remains on a No. 60 (250 m) sieve, and about 10 w/w% or less of the entirety of which passes through a No. 282 (53 µm) sieve. The specific volume of the "core" is about 5 ml/g or less, preferably about 3 ml/g or less.
Examples of the "core" include (1) a spherical granule of crystalline cellulose and lactose, (2) a spherical granule having a size of about 150 to 250 µm of crystalline cellulose (manufactured by Asahi Kasei Corporation, Avicel SP), (3) a granule having a size of about 50 to 250 µm produced from lactose (9 parts) and α-starch (1 part) by stirring granulation, (4) a micro particle having a size of about 250 µm or smaller obtained by classification of microcrystalline cellulose spherical granules described in JP-A-S61-213201, (5) a processed product of wax which is formed into a sphere by spray chilling or melt granulation, (6) a processed product such as a gelatin bead comprising an oil ingredient, (7) calcium silicate, (8) starch, (9) a porous particle such as chitin, cellulose, chitosan or the like, (10) a bulk powder of granulated sugar, crystalline lactose, crystalline cellulose, sodium chloride or the like, and a processed preparation thereof. Further, these cores may be produced by a per se known grinding method or granulation method, and then sieved to prepare particles having the desired particle diameter.

Examples of the "spherical granule of crystalline cellulose and lactose" include (i) a spherical granule having a size of 100 to 200 µm produced from crystalline cellulose (3 parts) and lactose (7 parts) (e.g., Nonpareil 105 (70-140) (particle diameter: about 100 to 200 µm), manufactured by Freund Corporation), (ii) a spherical granule having a size of about 150 to 250 µm produced from crystalline cellulose (3 parts) and lactose (7 parts) (e.g., Nonpareil NP-7:3, manufactured by Freund Corporation), (iii) a spherical granule having a size of about 100 to 200 µm produced from crystalline cellulose (4.5 parts) and lactose (5.5 parts) (e.g., Nonpareil 105T (70-140) (particle diameter: 100 to 200 µm), manufactured by Freund Corporation), (iv) a spherical granule having a size of about 150 to 250 µm produced from crystalline cellulose (5 parts) and lactose (5 parts) (e.g., Nonpareil NP-5:5, manufactured by Freund Corporation) and the like.
In order to produce a preparation retaining a suitable strength and having excellent solubility, the "core" is preferably a spherical granule produced from crystalline cellulose and lactose, and more preferably a spherical granule produced from crystalline cellulose and lactose which contains 50 wt% or more of lactose. A spherical granule composed of 40 - 50 wt%, of crystalline cellulose and 50 - 60 wt%, of lactose is preferable.
As the core to be used in the present invention, a spherical granule produced from crystalline cellulose and lactose is preferable, and an about 100 - 200 µm spherical granule produced from crystalline cellulose (4.5 parts) and lactose (5.5 parts) is more preferable.
While the "core" may contain PPI, since a coating layer comprising said PPI can control releaseability of the PPI, the core may not contain PPI.
The core may be fine granules, and is preferably as uniformly spherical as possible so as to minimize variation of coating amount.

The ratio of the "coating layer" to the "core" can be selected from the range enabling control of the dissolution property of the physiologically active substance and the particle size of the composition, and is, for example, generally about 50 - 400 parts by weight per 100 parts by weight of the core.
The coating layer may be formed by a plurality of coating layers, and at least one of the plural coating layers only needs to contain PPI. A combination of various coating layers, such as a coating layer free of the active ingredient, coating layer for base, enteric coating layer and the like, constituting the plural coating layers can be appropriately selected.
For coating of the core, for example, a mixture of the PPI and water-soluble polymer is used. The mixture may be a solution or a dispersion, which can be prepared using water or an organic solvent such as ethanol and the like, or a mixture thereof.
While the concentration of the water-soluble polymer in the mixture varies depending on the proportion of the PPI and the additive, it is generally about 0.1 - 50 wt%, preferably about 0.5 - 10 wt%, so as to maintain the binding force of the PPI to the core, as well as to maintain the viscosity of the mixture to prevent decreased workability.

When the coating layer comprises a plurality of layers, the concentration of the PPI in each layer may be changed successively or gradually by selecting the content or the viscosity grade of the water-soluble polymer of by coating successively using mixtures which are different in the proportions of the PPI and the other additives in the mixtures. In this case, coating may be performed using a mixture comprising the water-soluble polymer in an amount out of the range of about 0.1 to 50 wt%, as long as coating layers in total contain about 0.1 to 50 wt% of the water-soluble polymer. Further, the coating layer comprising a plurality of layers may comprise inert film layers formed by a known method so that the inert film layer can block each layer comprising the PPI.
The coated product is dried, and then passed through a sieve to obtain a composition having uniform particle size. The shape of the composition usually corresponds to the core, and thus a nearly spherical composition can be obtained. As the sieve, for example, a No. 50 (300 µm) round sieve can be used. The composition is obtained by screening from particles which pass through the No. 50 round sieve.

The "enteric fine granules containing PPI" is produced by coating a composition containing PPI with an enteric coating layer according to a granulation method similar to the above-mentioned method for the purpose of protecting PPI or conferring enteric property. If necessary, the fine granule may be further coated with a water-soluble sugar alcohol (preferably mannitol). When coated with a water-soluble sugar alcohol, the strength of an orally disintegrating tablet comprising the fine granules is improved.
The enteric coating layer is preferably a layer having a thickness of about 20 - 70 µm, preferably about 30 - 50 µm, which covers the whole surface of the composition containing PPI. Therefore, when the particle diameter of the composition is smaller, the weight percent of the enteric coating layer in the whole fine granules is higher. The enteric coating layer in the "enteric fine granules containing PPI" is about 30 to 70 wt%, preferably about 50 to 70 wt% of the whole fine granules.
The enteric coating layer may be composed of a plurality of layers (e.g., 2 to 3 layers). An example of a coating method comprises coating a composition with an enteric coating layer comprising polyethylene glycol, with an enteric coating layer comprising triethyl citrate, and then with an enteric coating layer comprising polyethylene glycol.

### (1)-3: "enteric fine granule-containing layer comprising PPI"

The "enteric fine granule-containing layer comprising PPI" of the multi-layer orally disintegrating tablet of the present invention is 1) a fine granule having an average particle size of about not more than 400 µm, which comprises a composition containing PPI, which is coated with an enteric coating layer and 2) an additive. The additive is a component contained in a part other than the enteric fine granules.
The amount of the "enteric fine granule-containing PPI" in the "enteric fine granule-containing layer comprising PPI" is about 20 - about 80 parts by weight, preferably about 30 - about 70 parts by weight, per 100 parts by weight of the "enteric fine granule-containing layer comprising PPI".
As the "additive", for example, one or more kinds selected from antacid, water-soluble sugar alcohol, crystalline cellulose, low-substituted hydroxypropylcellulose and the like is/are used, and further, binder, acidulant, foaming agent, sweetener, flavor, lubricant, colorant, stabilizer, excipient, disintegrant and the like is/are also used.
The orally disintegrating tablet of the present invention desirably contains antacid in a part other than enteric fine granules.
As the above-mentioned antacid, at least one kind (preferably, 1 - 15 kinds, more preferably, 1 - 10 kinds) of component selected from the group consisting of metal oxide, metal hydroxide, alkaline earth metal carbonate and aluminum glycinate is preferable.
As the above-mentioned metal oxide, at least one kind (preferably, 1 - 3 kinds) selected from the group consisting of magnesium oxide, magnesium silicate (2MgO·3SiO₂·xH₂O), dried aluminum hydroxide gel (Al₂O₃·xH₂O) and magnesium aluminometasilicate (Al₂O₃·MgO·2SiO₂·xH₂O), each for medicament, is preferably used.
Of these, magnesium oxide is more preferable. As magnesium oxide, one usable for medical purposes, superior in acid reactivity, and having a neutralizing ability is preferable. As such magnesium oxide, one obtained by a general production method or a commercially available product can be used, with preference given to magnesium oxide calcined at a low temperature, i.e., light burned magnesia. Magnesium oxide calcined at about 500 - about 1000°C is generally preferable, magnesium oxide calcined at about 600 - about 900°C is particularly preferable, and magnesium oxide calcined at about 800°C is most preferable, from the aspect of neutralizing ability. Of such magnesium oxide, one having a BET specific surface area of generally 10 - 50 m²/g, preferably 20 - 50 m²/g, is most preferable.
Here, the BET specific surface area refers to a specific surface area measured by a nitrogen gas adsorption method, wherein the specific surface area is determined from the adsorption amount of nitrogen gas on the surface of a given amount of magnesium oxide, including fine pores into which nitrogen gas enters.
Examples of the magnesium oxide include commercially available heavy magnesium oxide (manufactured by Kyowa Chemical Industries Ltd.), heavy magnesium oxide (manufactured by Tomita Pharmaceutical Co., Ltd.), heavy 30 magnesium oxide (manufactured by Kyowa Chemical Industries Ltd.), light magnesium oxide (manufactured by Kyowa Chemical Industries Ltd.) and the like. Particularly, heavy 30 magnesium oxide (manufactured by Kyowa Chemical Industries Ltd.) and the like are preferable.

Examples of the metal hydroxide include at least one kind (preferably, 1 - 3 kinds) selected from the group consisting of magnesium hydroxide, aluminum hydroxide, synthetic hydrotalcite (Mg₆Al₂(OH)₁₆CO₃·4H₂O), coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate, each for medicament, is preferable. Of these, magnesium hydroxide is preferable in terms of disintegration property of the preparation, dissolution property of PPI and the like.

Examples of the carbonate of the above-mentioned alkaline earth metal include calcium carbonate, magnesium carbonate and the like, each for medicament. Particularly, magnesium carbonate is preferable.

The above-mentioned metal oxide, metal hydroxide, alkaline earth metal carbonate and aluminum glycinate may be used alone or two or more kinds (preferably, 2 - 3 kinds) thereof may be combined.
As the antacid, a mixture of alkaline earth metal carbonate and aluminum glycinate is preferable, and a mixture of magnesium carbonate and aluminum glycinate is particularly preferable.
Some of metal oxide and metal hydroxide polish the surface of a formulating device during production to entirely or partially darken the surface of a tablet, form a dark spot, line or plane or attach to a punch during tableting. Since such property markedly impairs producibility, when metal oxide or metal hydroxide having abradability or punch-sticking property is to be selected, the polishing action and punch-sticking property can be suppressed by using a combination of metal oxide and metal hydroxide free of such property, or performing wet or dry granulation together with an additive (e.g., the below-mentioned excipient, binder, disintegrant and the like) usable as a pharmaceutical product.
In addition, antacid can contribute to the stabilization of aspirin by decreasing the contact with aspirin, by performing, in advance, wet or dry granulation together with an additive (e.g., the below-mentioned excipient, binder and the like).

The content of the above-mentioned antacid varies depending on the gastric acid neutralizing potency of each antacid, and is 0 mg - about 200 mg, preferably about 10 mg - about 100 mg, in the orally disintegrating tablet of the present invention.
In addition, the antacid is generally added at 0 - 50 parts by weight, preferably 10 - 40 parts by weight, per 100 parts by weight of the components other than the "enteric fine granule comprising PPI" in the "enteric fine granule-containing layer comprising PPI".

The aforementioned term "water-soluble sugar alcohol" means a sugar alcohol which requires less than 30 ml of water for dissolution within about 30 minutes when 1 g of the sugar alcohol is added to water and then strongly shaken at 20°C for 30 seconds every 5 minutes.
Examples of the "water-soluble sugar alcohol" include sorbitol, mannitol, maltitol, reduced starch saccharides, xylitol, reduced paratinose, erythritol and the like. Two or more kinds (preferably, 2 - 3 kinds) of these may be used in a mixture at an appropriate ratio.
The "water-soluble sugar alcohol" is preferably mannitol, xylitol or erythritol, more preferably mannitol or erythritol, particularly preferably mannitol. Erythritol is generally produced by fermentation of glucose as a starting material with yeast or the like. In the present invention, erythritol having a particle size of 50 mesh or less is used. The erythritol is commercially available (Nikken Chemicals Co., Ltd., etc.).
The "water-soluble sugar alcohol" is generally used at about 5 - 97 parts by weight, preferably about 10 - 90 parts by weight, per 100 parts by weight of the components other than the "enteric fine granule comprising PPI" in the "enteric fine granule-containing layer comprising PPI" to obtain sufficient strength of the preparation and sufficient oral disintegration property.

The aforementioned "crystalline cellulose" may be obtained by partial depolymerization of α-cellulose followed by purification. The "crystalline cellulose" also includes microcrystalline cellulose. Specific examples of the crystalline cellulose include Ceolus KG 801, Avicel PH 101, Avicel PH 102, Avicel PH 301, Avicel PH 302, Avicel RC-591 (crystalline cellulose/carmellose sodium) and the like. Preferred is so-called high-compatible Avicel including Ceolus KG 801. These crystalline celluloses may be used alone, or two or more kinds (preferably 2 or 3 kinds) may be used in combination. These crystalline celluloses are commercially available (Asahi Kasei Corporation).
The crystalline cellulose only needs to be added at about 3 - 50 parts by weight, preferably about 5 - 40 parts by weight, most preferably about 5 - 20 parts by weight, per 100 parts by weight of the components other than the "enteric fine granule comprising PPI" in the "enteric fine granule-containing layer comprising PPI".

The aforementioned "low-substituted hydroxypropylcellulose" means low-substituted hydroxypropylcellulose wherein the content of hydroxypropoxyl group in hydroxypropylcellulose (hereinafter sometimes to be abbreviated as HPC group content) is about 5.0 - 9.9 wt%. Particularly, it means low-substituted hydroxypropylcellulose of about 5.0 - 7.0 wt%, low-substituted hydroxypropylcellulose of about 7.0 - 9.9 wt% and the like.
Examples of the low-substituted hydroxypropylcellulose having an HPC group content of about 7.0 - 9.9% include LH-22, LH-32, a mixture thereof and the like, which are available as commercially available products (Shin-Etsu Chemical Co., Ltd.). Also, they can also be produced by a method known per se, for example, the method described in JP-B-S57-53100 as mentioned below or a method analogous thereto.
Examples of the low-substituted hydroxypropylcellulose having an HPC group content of about 5.0 - 7.0% include LH-23, LH-33, a mixture thereof and the like. These can be produced by a method known per se, for example, the method described in JP-B-S57-53100 or a method analogous thereto.
The particle size of the "low-substituted hydroxypropylcellulose having a hydroxypropoxyl group content of 5.0 - 7.0 wt%" is, for example, about 5 - 60 µm, preferably about 10 - 40 µm, as an average particle size.
Using L-HPC having a relatively large particle size (e.g., L-HPC having an average particle size of about 26 - 40 µm) from among such ranges, a preparation superior in the disintegration property can be produced. On the other hand, using L-HPC having a relatively small particle size (e.g., L-HPC having an average particle size of about 10 - 25 µm), a preparation superior in the preparation strength can be produced.
Thus, the particle size of L-HPC can be appropriately selected according to the characteristic of the desired preparation.
The low-substituted hydroxypropylcellulose having an HPC group content of 5.0 - 7.0 wt% and the low-substituted hydroxypropylcellulose having an HPC group content of about 7.0 - 9.9% are generally used at 0 - about 50 parts by weight, preferably 0 - about 40 parts by weight, more preferably 0 - about 20 parts by weight, per 100 parts by weight of the components other than the "enteric fine granule comprising PPI" in the "enteric fine granule-containing layer comprising PPI" to obtain sufficient oral disintegration property and sufficient strength of the preparation.

Example of the aforementioned "binder" include hydroxypropylcellulose, hydroxypropylmethylcellulose, crystalline cellulose, pregelatinized starch, polyvinylpyrrolidone, gum arabic powder, gelatin, pullulan, low-substituted hydroxypropylcellulose and the like. When crystalline cellulose is used as the binder, a solid preparation having a higher strength and retaining an excellent orally disintegrating property can be obtained.
Examples of the aforementioned "acidulant" include citric acid (anhydrous citric acid), tartaric acid, malic acid and the like.
Examples of the aforementioned "forming agent" include alkali metal carbonate (e.g., sodium carbonate, potassium carbonate and the like), alkali metal hydrogen carbonate (e.g., sodium hydrogen carbonate, potassium hydrogen carbonate and the like) and ammonium carbonate and the like.
Examples of the aforementioned "artificial sweetener" include saccharine sodium, dipotassium glycyrrhizinate, aspartame, stevia, thaumatin and the like.
The aforementioned "flavor" may be synthetic or natural, and examples thereof include lemon, lime, orange, menthol, strawberry and the like.
Examples of the aforementioned "lubricant" include magnesium stearate, a sucrose ester of fatty acid, polyethylene glycol, talc, stearic acid, hydrogenated oil and the like.
Examples of the aforementioned "colorant" include edible dyes such as food Yellow No. 5, food Red No. 2, and food Blue No. 2; an edible lake dye, red ferric oxide and the like.
Examples of the aforementioned "stabilizer" include the aforementioned basic inorganic salt and the like.
Examples of the aforementioned "excipient" include lactose, sucrose, D-mannitol, starch, corn starch, crystalline cellulose, light anhydrous silicic acid, titanium oxide and the like.

As the aforementioned "disintegrant", a disintegrant conventionally used in the pharmaceutical field can be used. Examples thereof include (1) crospovidone (e.g., crospovidone CL-F), (2) disintegrants referred to as super disintegrants such as croscarmellose sodium (FMC-Asahi Kasei Corporation) and carmellose calcium (GOTOKU CHEMICAL COMPANY LTD.), (3) sodium carboxymethyl starch (e.g., manufactured by Matsutani Chemical Industry Co., Ltd.), (4) low-substituted hydroxypropylcellulose (e.g., manufactured by Shin-Etsu Chemical Co., Ltd.), (5) cornstarch, (6) magnesium aluminometasilicate (e.g., Neusilin UFL2) and the like. Particularly preferable disintegrant includes, for example, crospovidone and/or magnesium aluminometasilicate.
The "crospovidone" may be any of those called polyvinylpolypyrrolidone (PVPP) and 1-vinyl-2-pyrrolidinone homopolymer, and crosslinked polymer having the chemical name of 1-ethenyl-2-pyrrolidinone homopolymer. Specific examples include kollidon CL (manufactured by BASF), polyplusdone XL (manufactured by ISP), polyplusdone XL-10 (manufactured by ISP), polyplusdone INF-10 (manufactured by ISP) and the like. The molecular weight generally exceeds 1,000,000.
These disintegrants may be used alone, or two or more kinds of them may be used in combination. For example, crospovidone may be used alone or in combination with other disintegrants.
Such disintegrant is generally used in about 3 - about 20 parts by weight, preferably about 5 - about 18 parts by weight, per 100 parts by weight of the components other than the "enteric fine granule comprising PPI" in the "enteric fine granule-containing layer comprising PPI".
The "enteric fine granule comprising PPI" in the present invention may contain, for example, titanium oxide and the like as a masking agent.

A preferable embodiment of the "enteric fine granule-containing layer comprising PPI" in the present invention is a layer containing 1) fine granules wherein a composition comprising PPI is coated with an enteric coating layer (preferably fine granules having an average particle size of not more than about 400 µm), 2) an antacid in a part other than the enteric fine granules and 3) the aforementioned additive.
Particularly, it is a layer containing 1) fine granules wherein a composition comprising PPI is coated with an enteric coating layer (preferably fine granules having an average particle size of not more than about 400 µm), 2) granules containing an antacid and additive (i) in a part other than the enteric fine granules, and 3) additive (ii) in a part other than the enteric fine granules and the granules.
The additives (i) and (ii) are appropriately selected from the aforementioned water-soluble sugar alcohol, crystalline cellulose, low-substituted hydroxypropylcellulose, binder, acidulant, foaming agent, sweetener, flavor, lubricant, colorant, stabilizer, excipient, disintegrant and the like.
Of these, water-soluble sugar alcohols such as mannitol and the like; excipients such as crystalline cellulose and the like; and binders such as hydroxypropylcellulose and the like are preferable as additive (i).
As additive (ii), disintegrants such as crospovidone, magnesium aluminometasilicate and the like; excipients such as crystalline cellulose and the like; lubricants such as magnesium stearate, and the like, and the like are preferable.

### (2) "acetylsalicylic acid-containing layer"

The "acetylsalicylic acid-containing layer" in the multi-layer orally disintegrating tablet of the present invention contains 1) acetylsalicylic acid and 2) an additive.
The content of acetylsalicylic acid is about 70 - about 400 mg per tablet. The content of acetylsalicylic acid per tablet is about 250 - about 400 mg as a non-steroidal anti-inflammatory drug (NSAIDs) when the treatment of pain, fever and inflammation is mainly desired.
On the other hand, the content is about 70 - about 120 mg when the suppression of thrombus or embolus formation (antiplatelet therapy) in the diseases of brain blood vessel and circulatory organ region, and the like is desired.
As the aforementioned "additive", the aforementioned water-soluble sugar alcohol, crystalline cellulose, low-substituted hydroxypropylcellulose, binder, acidulant, foaming agent, artificial sweetener, flavor, lubricant, colorant, stabilizer, excipient and the like are used, and water-soluble sugar alcohol, acidulant, artificial sweetener, lubricant, excipient and the like are preferably used. Furthermore, a disintegrant such as carboxymethylcellulose (carmellose), croscarmellose sodium, crystalline cellulose, pregelatinized starch, gelatin and the like, and a fluidizer such as light anhydrous silicic acid, hydrated silicon dioxide, talc, stearic acid and the like are also used.
Of the aforementioned components, examples of water-soluble sugar alcohol include mannitol, xylitol and erythritol, and mannitol is particularly preferable.
The amount of the water-soluble sugar alcohol to be added is about 10 - about 100 parts by weight, preferably about 25 - about 85 parts by weight, per 100 parts by weight of the "acetylsalicylic acid-containing layer".
As the lubricant, hydrogenated oil is preferably used in view of the suppression of tableting trouble. Particularly, when magnesium stearate and the like are used as the lubricant, the stability of acetylsalicylic acid sometimes decreases and the tableting trouble may occur.
The amount of the lubricant is about 0.5 - about 10 parts by weight, preferably about 0.5 - about 7 parts by weight, per 100 parts by weight of the "acetylsalicylic acid-containing layer".
As the disintegrant, carboxymethylcellulose (carmellose) is preferably used to improve the stability of acetylsalicylic acid.
The amount of the disintegrant is about 1 - about 20 parts by weight, preferably about 5 - about 15 parts by weight, per 100 parts by weight of the "acetylsalicylic acid-containing layer".

Acetylsalicylic acid and additives may be homogeneously mixed as they are, or homogeneously mixed along with other additives by using a premixed product of acetylsalicylic acid and an excipient (e.g., acetylsalicylic acid:cornstarch=90:10, dry granulation product).
The acetylsalicylic acid to be used for the orally disintegrating tablet of the present invention is desirably free of enteric coating.

### (3) multi-layer orally disintegrating tablet

The tablet weight of the multi-layer orally disintegrating tablet is about 300 mg - about 800 mg. The weight ratio of the "enteric fine granule-containing layer comprising PPI" and "acetylsalicylic acid-containing layer" is desirably about 10:1 - about 1:10, preferably about 5:1 - about 1:5, more preferably about 3:1 - about 1:3.

The "orally disintegrating tablet" of the present invention is produced by a method conventionally used in the pharmaceutical field.

### [Production method 1]

A composition for constructing the "enteric fine granule-containing layer comprising PPI" can be produced by, for example, mixing the "enteric fine granules comprising PPI" and an "additive" by a method known per se (composition 1).

A composition for constructing the "acetylsalicylic acid-containing layer" can be produced by mixing acetylsalicylic acid and an "additive" by a method known per se, and directly granulating the mixture or, when desired, granulating the mixture by a method known per se (composition 2).

A multi-layer orally disintegrating tablet can be produced by tableting composition 1 and composition 2 by a tableting method known per se.

### [Production method 2]

In the "enteric fine granule-containing layer comprising PPI", when an antacid is added to the "additive", granules may be produced by once mixing 1) antacid and 2) additives such as excipient, binder, disintegrant and the like, granulating the mixture by a method such as fluidized bed granulation method, high-speed stirring granulation method and the like, and drying the granules when desired (composition 3).
The composition 3, the "enteric fine granules comprising PPI" and other additives (disintegrant, artificial sweetener, excipient, lubricant etc.) are mixed to produce a composition for constructing the "enteric fine granule-containing layer comprising PPI", and the composition is tableted together with the above-mentioned composition 2 to give a multi-layer orally disintegrating tablet.

The "mixing" is performed by a mixing method generally used, for example, mixing, kneading, granulation and the like. The "mixing" is performed using an apparatus, such as vertical granulator VG10 (manufactured by Powrex Corporation), a universal kneader (manufactured by HATA IRON WORKS CO., LTD.), a fluidized bed granulator LAB-1, FD-3S (manufactured by Powrex Corporation), a V-type mixer, a tumbler mixer and the like.

The "granulation method" in the above-mentioned production method includes, for example, rotary granulation method (e.g., centrifugal rotary granulation), fluidized bed granulation (e.g., rotary fluidized bed granulation, fluidized granulation, etc.), stirring granulation and the like. Among others, preferred is fluidized bed granulation method, more preferred is rotary fluidized bed granulation method.
Concrete example of the rotary granulation method includes a method using "CF apparatus" manufactured by Freund Corporation and the like. Concrete examples of the rotary fluidized bed granulation method include methods using "SPIR-A-FLOW", "multi plex" manufactured by Powrex Corporation, "New-Marumerizer" manufactured by Fuji Paudal Co., Ltd., and the like. The method for spraying the mixture can be suitably selected in accordance with the kind of granulator, and may be, for example, any one of a top spray method, a bottom spray method, a tangential spray method, and the like. Among others, a tangential spray method is preferred.

The "tableting" is performed by tableting at a pressure of 1 - 80 kN/cm², 5 - 50 kN/cm², preferably 15 - 40 kN/cm², using a single punch tableting machine (manufactured by KIKUSUI SEISAKUSHO LTD.), a rotary tableting machine (manufactured by KIKUSUI SEISAKUSHO LTD.), a multi-layer rotary tableting machine (manufactured by HATA IRON WORKS CO., LTD.) and the like. In the case of a rotary tableting machine, tableting can be performed at a normal rotation speed, for example, 3 - 40 min⁻¹, preferably 3 - 30 min⁻¹, more preferably 5 - 25 min⁻¹.
The form of the orally disintegrating tablet in the present invention desirably has a curved surface on the tableted surface from the aspect of tablet strength, and a flat surface may be contained in part.

The "drying" may be performed by any method generally used for drying preparations, such as vacuum drying, fluidized bed drying and the like. Preferred is drying by vacuum drying.

The method of tableting the orally disintegrating tablet of the present invention may be performed at room temperature or at a temperature exceeding the room temperature. The "room temperature" refers to the temperature in the room where tableting is performed for general production of tablets, which is generally about 20 - about 23°C. That is, the "temperature exceeding the room temperature" means a temperature higher than this temperature, and the lower limit is preferably about 25°C. While the temperature varies depending on the starting powder material and granules to be used, and the like, it is generally preferably about 25°C - about 50°C. The temperature can be changed according to the desired quality of the tablet.

The orally disintegrating tablet of the present invention is desirably a bi-layer tablet having the "enteric fine granule-containing layer comprising PPI" and the "acetylsalicylic acid-containing layer", and may have an intermediate layer between the "enteric fine granule-containing layer comprising PPI" and the "acetylsalicylic acid containing layer".
Since the intermediate layer blocks a direct contact between the "enteric fine granule-containing layer comprising PPI" and the "acetylsalicylic acid-containing layer", the stability of PPI and acetylsalicylic acid can be further improved.
Examples of the material for the above-mentioned intermediate layer include an appropriate mixture of a polymer substrate such as low-substituted hydroxypropylcellulose, hydroxypropylcellulose, hydroxypropylmethylcellulose (e.g., TC-5 and the like), polyvinylpyrrolidone, polyvinyl alcohol, methylcellulose, hydroxyethylmethylcellulose, etc. and the like, and saccharides such as sucrose (purified sucrose (pulverized (powder sugar) and unpulverized) and the like), starch saccharide (cornstarch and the like), lactose, honey and sugar alcohol (D-mannitol, erythritol and the like) and the like. Furthermore, the intermediate layer may contain, where necessary, excipients (e.g., masking agent (titanium oxide and the like), antistatic agents (titanium oxide, talc and the like)) and the like as appropriate.
The amount of the intermediate layer in the tablet is generally about 5 parts by weight - about 50 parts by weight, preferably about 10 - about 45 parts by weight, per tablet.
The intermediate layer can be formed by a conventional method.
The intermediate layer may be formed from not only one layer but also plural layers (preferably, 2 - 3 layers).

The "orally disintegrating tablet" of the present invention having a tablet diameter of about 5 - 20 mm, preferably about 7 - 15 mm, more preferably about 8 - 13 mm, facilitates handling for administration.
The "orally disintegrating tablet" of the present invention shows rapid oral disintegration property or dissolution property, and appropriate preparation strength.
The oral disintegration time (a time until an orally disintegrating tablet is completely disintegrated with saliva in the oral cavity of a healthy adult man or woman) of the orally disintegrating tablet of the present invention is within about 60 seconds, generally within about 50 seconds, preferably within about 40 seconds.
Furthermore, the disintegration time of the orally disintegrating tablet of the present invention (time until complete disintegration of the orally disintegrating tablet in a disintegration test performed at a solution temperature of 37±2°C using the disintegration test apparatus described in the Japanese Pharmacopeia and water as a test solution) is within about 90 seconds, preferably within about 60 seconds, most preferably within about 50 seconds.
In addition, the orally disintegrating tablet of the present invention has appropriate hardness of the level capable of avoiding damage in the preparation steps and distribution process, and the strength of the orally disintegrating tablet (measured values by a tablet hardness tester) is generally about 20 - about 100N, more preferably about 30 - about 90N.

The orally disintegrating tablet of the present invention is administered without water or together with water. Examples of an administration method include (1) a method comprising holding the preparation of the present invention in the mouth and not swallowing the preparation as it is, and then dissolving or disintegrating the preparation with a small amount of water or with saliva in the oral cavity without water or (2) a method comprising swallowing a preparation as it is together with water. Alternatively, the tablet of the present invention may be dissolved or disintegrated with water, and then be administered.

Since the orally disintegrating tablet of the present invention comprises PPI, it shows superior antiulcer activity, gastric acid secretion-inhibitory action, mucosa-protecting action, anti-Helicobacter pylori action and the like.
On the other hand, since the orally disintegrating tablet of the present invention contains acetylsalicylic acid, it is useful as an agent for the suppression of thrombus or embolus formation in the diseases of brain blood vessel and circulatory organ region, for example, angina pectoris (chronic stable angina pectoris, unstable angina pectoris) and myocardial infarction; the prophylaxis or treatment of ischemic cerebrovascular disorders (transient cerebral ischemic attack (TIA), cerebral infarction); suppression of thrombus or embolus formation after operation of coronary artery bypass graft (CABG) or percutaneous transluminal coronary angioplasty (PTCA); and an agent for the prophylaxis or treatment of Kawasaki disease (including cardiovascular sequelae due to Kawasaki disease). Therefore, the orally disintegrating tablet of the present invention can be administered for the purpose of the treatment or suppression of the onset of gastric ulcer or duodenal ulcer, while continuing aspirin medication. When the prophylaxis or treatment of such diseases is desired, PPI is administered at about 10 mg - about 40 mg per day, and acetylsalicylic acid is administered at (a low dose of) about 70 mg - about 120 mg per day.
In addition, acetylsalicylic acid can also be used as one kind of non-steroidal anti-inflammatory drugs (NSAIDs) mainly for the treatment of pain, fever and inflammation. NSAIDs sometimes cause gastric ulcer or duodenal ulcer. However, particularly, in the treatment of rheumatoid arthritis, osteoarthritis and the like, since QOL decreases markedly, discontinuation of NSAIDs administration is sometimes difficult. In such cases, the orally disintegrating tablet of the present invention can be administered for the purpose of treating or suppressing the onset of gastric ulcer or duodenal ulcer, while continuing the NSAIDs administration,
When such treatment is desired, the PPI is administered at about 10 mg - about 40 mg per day, and acetylsalicylic acid is administered at about 240 mg - about 400 mg per day.
Therefore, such orally disintegrating tablet of the present invention is useful as a low toxic and safe combination drug of PPI and acetylsalicylic acid.
The orally disintegrating tablet of the present invention can be orally administered to mammals (e.g., human, monkey, sheep, horse, dog, cat, rabbit, rat, mouse and the like) for the purpose of suppressing thrombus or embolus formation in the diseases of brain blood vessel and circulatory organ region, treating or preventing ulcer caused by non-steroidal anti-inflammatory agents; and the like.
In addition to the above-mentioned object, the orally disintegrating tablet of the present invention, and a penicillin antibiotic (e.g., amoxicillin and the like) and an erythromycin antibiotic (e.g., clarithromycin and the like) may be used in combination for bacterial elimination of Helicobacter pylori or aiding the bacterial elimination.
The daily dose of the solid preparation of the present invention varies depending on the level of symptom, age, sex, body weight of administration subject, administration term, interval, the kind of active ingredient and the like, and is not particularly limited. In addition, the orally disintegrating tablet of the present invention may be administered once a day or in 2 - 3 portions per day. Examples

The present invention is explained in more detail in the following by referring to Reference Examples, Examples and Evaluations (Experimental Examples), which are not to be construed as limitative. In the following, the formulation additives used in Reference Examples and Examples were the Japanese Pharmacopoeia products and Japanese Pharmaceutical Excipients products.

### Reference Example 1

### Production of lansoprazole enteric fine granules principal drug fine granules

Nonpareil 105 (trade name, 39.6 kg) was placed in a centrifugal fluidized bed coating granulator (manufactured by Powrex Corporation, MP-400), and a principal drug coating solution having the following composition and prepared in advance was sprayed for coating. Furthermore, an intermediate layer coating solution having the following composition and prepared in advance was sprayed for coating. After the completion of coating, the granules were dried to give principal drug fine granules (145.2 kg).

**[principal drug coating solution]**

| | |
|---|---|
| lansoprazole | 39.60 kg |
| magnesium carbonate | 13.20 kg |
| low-substituted hydroxypropylcellulose | 6.60 kg |
| hydroxypropylcellulose | 13.20 kg |
| (purified water) | (185 L) |

**[intermediate layer coating solution]**

| | |
|---|---|
| HPMC | 9.24 kg |
| low-substituted hydroxypropylcellulose | 6.60 kg |
| sterilization talc | 3.96 kg |
| titanium oxide | 3.96 kg |
| mannitol | 9.24 kg |
| (purified water) | (99.0 L) |

### Lansoprazole enteric fine granules

Principal drug fine granules (44.0 kg) were placed in a centrifugal fluidized bed coating-granulator (manufactured by Powrex Corporation, MP-400), and an enteric coating solution 1 having the following composition and prepared in advance was sprayed for coating. Then, an enteric coating solution 2 having the following composition and prepared in advance was sprayed for coating. Furthermore, an over-coating solution having the following composition and prepared in advance was sprayed for coating. After the completion of coating, the granules were dried to give Lansoprazole enteric fine granules (108 kg).

| [glycerol monostearate solution] | |
|---|---|
| glycerol monostearate | 3.150 kg |
| polysorbate 80 | 0.945 kg |
| yellow ferric oxide | 0.0315 kg |
| red ferric oxide | 0.0315 kg |
| (purified water) | (63 L) |

**[enteric coating solution 1]**

| | |
|---|---|
| Eudragit L30D-55 | 9.614 kg solid amount |
| | (32.05 kg) (liquid amount) |
| Eudragit NE30D | 1.071 kg solid amount |
| | (3.570 kg) (liquid amount) |
| polyethylene glycol 6000 | 1.071 kg |
| citric anhydride | 0.01260 kg |
| (purified water) | (31.8 L) |
| glycerol monostearate solution | 0.8316 kg solid amount |
| | (13.4 kg) (liquid amount) |

**[enteric coating solution 2]**

| | |
|---|---|
| Eudragit L30D-5 | 35.28 kg solid amount |
| | (117.6 kg) (liquid amount) |
| Eudragit NE30D | 3.919 kg solid amount |
| | (13.06 kg) (liquid amount) |
| triethyl citrate | 7.854 kg |
| citric anhydride | 0.02100 kg |
| (purified water) | (9.33 L) |
| glycerol monostearate solution | 3.3264 kg solid amount |
| | (53.7 kg) (liquid amount) |

### [over-coating solution]

| | |
|---|---|
| mannitol | 4.200 kg |
| (purified water) | (25.2 L) |

### Example 1

An aspirin premixed product (aspirin:cornstarch=90:10 dry granulation product, manufactured by Rhodia, Rhodine 2312, 1080 g), mannitol (manufactured by Roquette Pharma, PEARLITOL 200SD, 924 g), carmellose (240 g), citric anhydride (21.6 g), aspartame (24.0 g) and hydrogenated oil (110.4 g) were measured and mixed to give granule A. Aluminum glycinate (88.0 g), magnesium carbonate (176.0 g), mannitol (manufactured by Roquette Pharma, PEARLITOL 200SD, 403.3 g) and crystalline cellulose (47.7 g) were measured and placed in a fluidized bed dryer (POWREX CORPORATION, LAB-1), and the mixture was granulated while spraying a 5% hydroxypropylcellulose (HPC-L) solution (540.8 g) and dried to give granules. Furthermore, granules were obtained by repeating in the same manner and mixed with the granules produced earlier to give granule B. Granule B (1113.0 g), Lansoprazole enteric fine granules (1620 g), crospovidone (179.4 g), aspartame (32.4 g), crystalline cellulose (259.2 g, manufactured by Asahi Kasei, CEOLUS KG-1000), Neusilin (manufactured by Fuji Chemical Industries, trade name, 66.0 g) and magnesium stearate (30.0 g) were measured and mixed to give granule C. Granule A and granule C were tableted using a tableting machine (PICCOLA, manufactured by RIVA) at a tableting pressure of 11 kN to give a layered tablet having a diameter of 11 mm (preparation 1). The composition per tablet of this preparation 1 is shown in Table 1.

**Table 1**

| Blending composition of preparation 1 | |
|---|---|
| Component | preparation 1 |
| aspirin | 81 |
| cornstarch | 9.00 |
| mannitol | 77.0 |
| carmellose | 20.0 |
| citric anhydride | 1.8 |
| aspartame | 2.0 |
| hydrogenated oil | 9.2 |
| Granule A subtotal (mg) | 200.0 |
| aluminum glycinate | 11.0 |
| magnesium carbonate | 22.0 |
| mannitol | 50.41 |
| crystalline cellulose | 5.96 |
| HPC-L | 3.38 |
| Lansoprazole fine granules | 135 |
| crospovidone | 14.95 |
| aspartame | 2.70 |
| crystalline cellulose | 21.60 |
| Neusilin | 5.50 |
| magnesium stearate | 2.50 |
| Granule C subtotal (mg) | 275.00 |
| total (mg) | 475.00 |

### Example 2

An aspirin premixed product (aspirin:cornstarch=90:10 dry granulation product, manufactured by Rhodia, Rhodine 2312, 1800 g), mannitol (manufactured by Roquette Pharma, PEARLITOL 200SD, 1540 g), carmellose (400 g), citric anhydride (36.0 g), aspartame (40.0 g) and hydrogenated oil (184.0 g) were measured and mixed to give granule D. Mannitol (manufactured by Roquette Pharma, PEARLITOL 200SD, 604.9 g) and crystalline cellulose (71.5 g) were measured and placed in a fluidized bed dryer (POWREX CORPORATION, MP-01), and the mixture was granulated while spraying a 5% hydroxypropylcellulose solution (811.2 g) and dried to give granule E. Granule E (298.9 g), Lansoprazole enteric fine granules (675 g), crospovidone (74.75 g), aspartame (13.52 g), crystalline cellulose (108 g, manufactured by Asahi Kasei, CEOLUS KG-1000), Neusilin (manufactured by Fuji Chemical Industries, trade name, 27.48 g) and magnesium stearate (12.5 g) were measured and mixed to give granule F. Granule D and granule F were tableted using a tableting machine (PICCOLA, manufactured by RIVA) at a tableting pressure of 15 kN to give a layered tablet having a diameter of 11 mm (preparation 2). The composition per tablet of preparation 2 is shown in Table 2.

**Table 2**

| Blending composition of preparation 2 | |
|---|---|
| component | preparation 2 |
| aspirin | 81 |
| cornstarch | 9.00 |
| mannitol | 77.0 |
| carmellose | 20.0 |
| citric anhydride | 1.8 |
| aspartame | 2.0 |
| hydrogenated oil | 9.2 |
| Granule D subtotal (mg) | 200.0 |
| mannitol | 50.41 |
| crystalline cellulose | 5.96 |
| HPC-L | 3.38 |
| Lansoprazole fine granules | 135 |
| crospovidone | 14.95 |
| aspartame | 2.70 |
| crystalline cellulose | 21.60 |
| Neusilin | 5.50 |
| magnesium stearate | 2.50 |
| Granule F subtotal (mg) | 242.00 |
| total (mg) | 442.00 |

### Example 3

An aspirin premixed product (aspirin:cornstarch=90:10 dry granulation product, manufactured by Rhodia, Rhodine 2312, 900 g), mannitol (manufactured by Roquette Pharma, PEARLITOL 200SD, 760 g), carmellose (200 g), citric anhydride (17.93 g), aspartame (20.0 g), light anhydrous silicic acid (10.0 g) and hydrogenated oil (91.99 g) were measured and mixed to give granule G. Aluminum glycinate (550.3 g), magnesium carbonate (1100.2 g), mannitol (manufactured by Roquette Pharma, PEARLITOL 200SD, 2545.5 g) and crystalline cellulose (298.0 g) were measured and placed in a fluidized bed dryer (Freund Corporation, FLO-5M), and the mixture was granulated while spraying a 7% hydroxypropylcellulose solution (2414 g) and dried to give granules. Furthermore, granules were obtained by repeating in the same manner and mixed with the granules produced earlier to give granule H. Granule H (1091.7 g), Lansoprazole enteric fine granules (2025.2 g), crospovidone (224.3 g), aspartame (40.5 g), crystalline cellulose (323.8 g, manufactured by Asahi Kasei, CEOLUS KG-1000), Neusilin (manufactured by Fuji Chemical Industries, trade name, 82.5 g) and magnesium stearate (37.5 g) were measured and mixed to give granule I. Granule G and granule I were tableted using a tableting machine (PICCOLA, manufactured by RIVA) at a tableting pressure of 8.6 kN to give a layered tablet having a diameter of 10 mm (preparation 3). The composition per tablet of preparation 3 is shown in Table 3.

**Table 3**

| Blending composition of preparation 3 | |
|---|---|
| Component | preparation 3 |
| aspirin | 81 |
| cornstarch | 9.00 |
| mannitol | 76.0 |
| carmellose | 20.0 |
| citric anhydride | 1.8 |
| aspartame | 2.0 |
| light anhydrous silicic acid | 1.0 |
| hydrogenated oil | 9.2 |
| Granule G subtotal (mg) | 200.0 |
| aluminum glycinate | 11.0 |
| magnesium carbonate | 22.0 |
| mannitol | 50.41 |
| crystalline cellulose | 5.96 |
| HPC-L | 3.38 |
| Lansoprazole fine granules | 135 |
| crospovidone | 14.95 |
| aspartame | 2.70 |
| crystalline cellulose | 21.60 |
| Neusilin | 5.50 |
| magnesium stearate | 2.50 |
| Granule I subtotal (mg) | 275.00 |
| total (mg) | 475.00 |

### Example 4

Aspirin (1214.6 g), cornstarch (135.0 g), mannitol (manufactured by Roquette Pharma, PEARLITOL 200SD, 1140.2 g), carmellose (300.0 g), citric anhydride (27.0 g), aspartame (30.0 g), light anhydrous silicic acid (14.93 g) and hydrogenated oil (138.2 g) were measured and mixed to give granule J. Granule J and the above-mentioned granule I were tableted using a tableting machine (PICCOLA, manufactured by RIVA) at a tableting pressure of 10 kN to give a layered tablet having a diameter of 10.5 mm (form: 2 step R) (preparation 4). The composition per tablet of preparation 4 is shown in Table 4.

**[Table 4]**

| Blending composition of preparation 4 | |
|---|---|
| component | preparation 4 |
| aspirin | 81 |
| cornstarch | 9.00 |
| mannitol | 76.0 |
| carmellose | 20.0 |
| citric anhydride | 1.8 |
| aspartame | 2.0 |
| light anhydrous silicic acid | 1.0 |
| hydrogenated oil | 9.2 |
| Granule J subtotal (mg) | 200.0 |
| aluminum glycinate | 11.0 |
| magnesium carbonate | 22.0 |
| mannitol | 50.41 |
| crystalline cellulose | 5.96 |
| HPC-L | 3.38 |
| Lansoprazole fine granules | 135 |
| crospovidone | 14.95 |
| aspartame | 2.70 |
| crystalline cellulose | 21.60 |
| Neusilin | 5.50 |
| magnesium stearate | 2.50 |
| Granule I subtotal (mg) | 275.00 |
| total (mg) | 475.00 |

### Example 5

An aspirin premixed product (aspirin: cornstarch=90:10 dry granulation product, manufactured by Rhodia, Rhodine 2312, 31500 g), mannitol (manufactured by Roquette Pharma, PEARLITOL 200SD, 28420 g), carmellose (7000 g), citric anhydride (630 g), aspartame (700 g), light anhydrous silicic acid (manufactured by NIPPON AEROSIL, AEROSIL, 350 g) and hydrogenated oil (1400 g) were measured and mixed by a tumbler mixer to give granule K. Aluminum glycinate (4840 g), magnesium carbonate (9680 g), mannitol (manufactured by Roquette Pharma, PEARLITOL 200SD, 22180 g), crystalline cellulose (manufactured by Asahi Kasei, CEOLUS PH-101, 2622 g), crospovidone (6578 g), aspartame (1188 g), crystalline cellulose (9504 g, manufactured by Asahi Kasei, CEOLUS KG-1000) and Neusilin (manufactured by Fuji Chemical Industries, trade name, 2420 g) were measured and placed in a fluidized bed dryer (POWREX CORPORATION, WSG-60 type), and the mixture was granulated while spraying a 6% hydroxypropylcellulose solution (24785 g) and dried to give granule L. Granule L was sieved by a power mill, 34380 g thereof was measured and mixed with Lansoprazole enteric fine granules (33750 g) and magnesium stearate (625 g) by a tumbler mixer to give granule M. Granule K and granule M were tableted using a tableting machine (manufactured by HATA IRON WORKS CO., LTD., HT-CVX54LS-UW/C&3L) at a tableting pressure of 11 kN to give a layered tablet having a diameter of 10.5 mm. This layered tablet was vacuum-dried to give preparation 5. The composition per tablet of preparation 5 is shown in Table 5.

**[Table 5]**

| Blending composition of preparation 5 | |
|---|---|
| Component | preparation 5 |
| aspirin | 81 |
| cornstarch | 9.00 |
| mannitol | 81.2 |
| carmellose | 20.0 |
| citric anhydride | 1.8 |
| aspartame | 2.0 |
| light anhydrous silicic acid | 1.0 |
| hydrogenated oil | 4.0 |
| Granule K subtotal (mg) | 200.0 |
| aluminum glycinate | 11.0 |
| magnesium carbonate | 22.0 |
| mannitol | 50.41 |
| crystalline cellulose (PH101) | 5.96 |
| HPC-L | 3.38 |
| crospovidone | 14.95 |
| aspartame | 2.70 |
| crystalline cellulose (KG1000) | 21.6 |
| Neusilin | 5.50 |
| Lansoprazole fine granules | 135 |
| magnesium stearate | 2.50 |
| Granule M subtotal (mg) | 275.00 |
| total (mg) | 475.00 |

### Evaluation

### Experimental Example 1

The tablets obtained in Example 1 and Example 5, after storage in the closed bottle with a desiccant at 40°C for 1 month, were quantified for the content of aspirin in the preparation by high performance liquid chromatography. The residual ratio (%) of aspirin (=aspirin content after storage/initial aspirin content×100) was calculated from the quantified value. For evaluation, an aspirin residual ratio of not less than 95% and not more than 100% was marked with ○, not less than 90% and less than 95% with Δ, and less than 90% with x. The evaluation results are shown in Table 6.

**[Table 6]**

| Aspirin stability test results | |
|---|---|
| Example 1 | ○ |
| Example 5 | ○ |

### Experimental Example 2

The tablets obtained in Example 1 and Example 5 were measured for the oral disintegration time. The time from placing the tablet in the mouth of a healthy individual to the dissolution thereof without biting at all was taken as the oral disintegration time (average of n=2). The results are shown in Table 7.

**[Table 7]**

| Oral disintegration time measurement results | |
|---|---|
| Example 1 | 37 seconds |
| Example 5 | 43 seconds |

### Experimental Example 3

Using a tablet measurement apparatus (manufactured by PHARMA TEST), the tablets obtained in Example 1 and Example 5 were measured for the initial tablet hardness. The test was performed 5 times for Example 1 and 10 times for Example 5 and the average values thereof are shown in Table 8.

**[Table 8]**

| Tablet hardness measurement results | |
|---|---|
| Example 1 | 61N |
| Example 5 | 56N |

### Industrial Applicability

Since the orally disintegrating tablet of the present invention contains PPI having a strong acid secretion-inhibitory action and acetylsalicylic acid, which is one kind of NSAIDs, it can be administered for treating or suppressing the onset of gastric ulcer or duodenal ulcer while continuing the NSAIDs administration. In addition, the orally disintegrating tablet of the present invention shows high stability of the active ingredients, and expresses the pharmacological effect of the active ingredients stably and rapidly after administration. Furthermore, the orally disintegrating tablet of the present invention is convenient since it can be administered on demand easily, anytime and anywhere, without water.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

The present invention is based on JP2010-270276, the contents of which are encompassed in full herein.

## Claims

1. A multi-layer orally disintegrating tablet comprising (1) an enteric fine granule-containing layer comprising a proton pump inhibitor and (2) an acetylsalicylic acid-containing layer.

2. The orally disintegrating tablet according to claim 1, wherein the enteric fine granule-containing layer comprises an antacid in a part other than the enteric fine granules.

3. The orally disintegrating tablet according to claim 2, wherein the antacid is at least one kind of component selected from the group consisting of metal oxide, metal hydroxide, alkaline earth metal carbonate and aluminum glycinate.

4. The orally disintegrating tablet according to claim 3, wherein the metal oxide is at least one kind selected from the group consisting of magnesium oxide, magnesium silicate, dried aluminum hydroxide gel and magnesium aluminometasilicate.

5. The orally disintegrating tablet according to claim 3, wherein the metal hydroxide is at least one kind selected from the group consisting of magnesium hydroxide, aluminum hydroxide, synthetic hydrotalcite, coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate.

6. The orally disintegrating tablet according to claim 3, wherein the alkaline earth metal carbonate is calcium carbonate or magnesium carbonate.

7. The orally disintegrating tablet according to claim 2, wherein the antacid is a mixture of magnesium carbonate and aluminum glycinate.

8. The orally disintegrating tablet according to claim 2, wherein the content of the antacid is about 10 mg - about 100 mg.

9. The orally disintegrating tablet according to claim 1, wherein the proton pump inhibitor is lansoprazole, omeprazole, rabeprazole, pantoprazole or an optically active form thereof or a salt thereof.

10. The orally disintegrating tablet according to claim 1, wherein the content of the acetylsalicylic acid is about 70 mg - about 120 mg per tablet.

11. The orally disintegrating tablet according to claim 1, wherein the acetylsalicylic acid-containing layer comprises carboxymethylcellulose.

12. The orally disintegrating tablet according to claim 1, wherein the enteric fine granule-containing layer comprising a proton pump inhibitor comprises at least one kind of a disintegrant selected from crospovidone and magnesium aluminometasilicate in a part other than the enteric fine granules.

13. The orally disintegrating tablet according to claim 1, wherein the acetylsalicylic acid-containing layer comprises a lubricant.

14. The orally disintegrating tablet according to claim 13, wherein the lubricant is hydrogenated oil.

15. The orally disintegrating tablet according to claim 1, which is a bi-layer tablet.

16. The orally disintegrating tablet according to claim 1, comprising an intermediate layer between (1) the enteric fine granule-containing layer comprising a proton pump inhibitor and (2) the acetylsalicylic acid-containing layer.

17. The orally disintegrating tablet according to claim 1, wherein the acetylsalicylic acid is not enteric-coated.

18. The orally disintegrating tablet according to claim 1, wherein the tablet weight is about 300 mg - about 800 mg, and the weight ratio of (1) the enteric fine granule-containing layer comprising a proton pump inhibitor and (2) the acetylsalicylic acid-containing layer is about 10:1 - about 1:10.

19. The orally disintegrating tablet according to claim 1, having a curved surface on a tablet surface.

20. The orally disintegrating tablet according to claim 1, having an oral disintegration time of within about 60 seconds.

21. The orally disintegrating tablet according to claim 1, having a tablet hardness of about 20 - about 100 N.
